# FASCICULE DE BREVET EUROPEEN

(11) **EP 0 693 059 B1**
(45) Date de publication et mention de la délivrance du brevet: **12.03.1997**
(21) Numéro de dépôt: 94912582.7
(22) Date de dépôt: 01.04.1994
(51) Int. Cl.: C07D 209/44, C07D 403/06, C07D 403/10, A61K 31/40

(54) **DERIVES DE PERHYDROISOINDOLE, LEUR PREPARATION ET LES COMPOSITIONS PHARMACEUTIQUES QUI LES CONTIENNENT**
PERHYDROISOINDOL-DERIVATE, DEREN HERSTELLUNG UND DIE SIE BEHALTENDE PHARMAZEUTISCHE ZUSAMMENFASSUNGEN
PERHYDROISOINDOLE DERIVATIVES, PREPARATION THEREOF AND PHARMACEUTICAL COMPOSITIONS CONTAINING SAME

(30) Priorité: 05.04.1993 FR 9303965
(43) Date de publication de la demande: 24.01.1996
(73) Titulaire: RHONE-POULENC RORER S.A., 92160 Antony (FR)
(72) Inventeur: CRESPO, André, F-94490 Ormesson (FR); FARDIN, Véronique, F-94100 Saint-Maur (FR); GUILLAUME, Jean-Marc, F-75011 Paris (FR); MALLERON, Jean-Luc, F-91460 Marcoussis (FR); PEYRONEL, Jean-François, F-91120 Palaiseau (FR)
(74) Mandataire: Savina, Jacques
(86) Numéro de dépôt international: FR9400371
(87) Numéro de publication internationale: WO9422822

(56) Documents cités:
- EP-A- 0 429 366
- EP-A- 0 430 771
- WO-A-92/20653
- WO-A-92/20654
- WO-A-93/21154
- WO-A-93/21155

## Description

La présente invention concerne des dérivés du perhydroisoindole de formule générale : 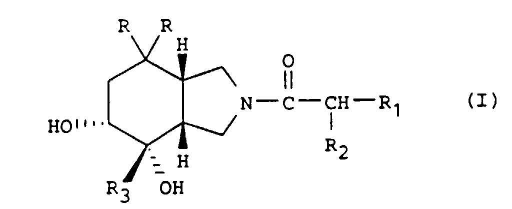 ainsi que leurs sels lorsqu'ils existent, qui antagonisent les effets de la neurokinine A et sont de ce fait particulièrement intéressants dans les domaines thérapeutiques où cette substance est connue pour intervenir.

La neurokinine A est impliquée dans de nombreuses pathologies telles que la transmission de la douleur, l'arthrite, l'asthme, les phénomènes inflammatoires, les psychoses, les désordres tensionnels, les désordres vésicaux, les cystites ..., c'est pourquoi les dérivés de l'isoindole de formule générale (I) présentent un intérêt considérable.

Les effets de la neurokinine A sont médiés principalement par les récepteurs NK2.

Dans la demande de brevet européen EP 429 366 ont été décrits des antagonistes de la substance P de structure : 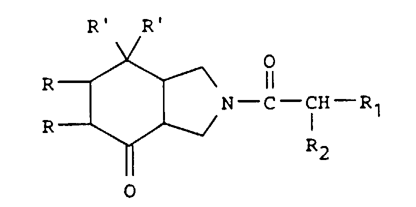 dans laquelle les symboles R sont hydrogène ou forment ensemble une liaison, les symboles R' sont des radicaux phényle éventuellement substitués et les symboles R₁ et R₂ représentent diverses substitutions. Cependant ces dérivés de perhydroisoindolone ne manifestent pas d'activité antagoniste des récepteurs NK2.

Dans le brevet américain 4 042 707 avaient été décrits des produits dérivés de l'isoindole de formule générale: 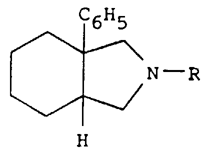 ayant une activité opiacée. Ces produits n'ont pas d'activité vis-à-vis de la neurokinine A.

Dans la formule générale (I) :
- les symboles R sont identiques et représentent des radicaux phényle éventuellement substitués par un atome d'halogène ou par un radical méthyle en position 2 ou 3,
- le symbole R₁ représente un radical phényle éventuellement substitué par un ou plusieurs atomes d'halogène ou radicaux hydroxy, benzyloxy, alcoyle pouvant être éventuellement substitués (par des atomes d'halogène ou des radicaux amino, alcoylamino ou dialcoylamino), alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle, alcoyloxy, aminoalcoyle, alcoylaminoalcoyle ou dialcoylaminoalcoyle,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino, et
- le symbole R₃ représente un radical phényle éventuellement substitué en position -2 par un radical alcoyle ou alcoyloxy contenant 1 ou 2 atomes de carbone.

Il est entendu que les radicaux alcoyle ou acyle cités ci-dessus contiennent (sauf mention spéciale) 1 à 4 atomes de carbone en chaîne droite ou ramifiée.

Lorsque R porte un substituant halogène, ce dernier peut être choisi parmi le chlore ou le fluor.

Lorsque R₁ contient un atome d'halogène, ce dernier peut être choisi parmi le chlore, le brome, le fluor ou l'iode.

Lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle.

Lorsque R₁ représente phényle substitué par une chaîne portant un hétérocycle, ce dernier peut être choisi parmi pyrrolidinyle, morpholino, pipéridinyle, tétrahydropyridinyle, pipérazinyle, thiomorpholino.

Lorsque le symbole R₂ est autre que l'atome d'hydrogène, la chaîne substituée sur l'isoindole présente un centre chiral, il est entendu que les formes stéréoisomères (R) ou (S) et leurs mélanges font partie de la présente invention.

Par ailleurs, les formes racémiques (3aRS,4RS,5RS,7aRS) des dérivés de perhydroisoindole de formule générale (I) manifestent également une activité antagoniste de la neurokinine A. Leur application à la préparation d'un médicament destiné aux traitements des affections médiées par le récépteur à tachykinines NK2, entrent aussi dans le cadre de la présente invention.

Selon l'invention les dérivés du perhydroisoindole de formule générale (I) peuvent être obtenus par action de l'acide de formule générale : 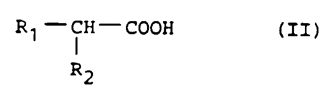 ou d'un dérivé réactif de cet acide, dans lequel R₁ et R₂ sont définis comme précédemment, sur un dérivé de l'isoindole de formule générale : 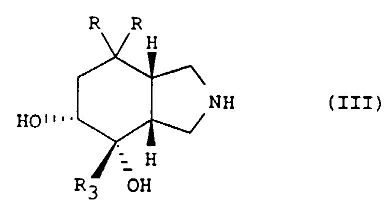 dans laquelle les symboles R et R₃ sont définis comme précédemment.

Il est entendu que, les radicaux amino, alcoylamino ou carboxy contenus dans R₁ et/ou R₂ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R₂ représente un radical hydroxy, il est préférable de protéger préalablement ce radical. La protection s'effectue par exemple par un radical acétyle, trialcoylsilyle, benzyle, sous forme d'un carbonate par un radical -COORa dans lequel Ra est un radical alcoyle ou benzyle ou sous forme de cétone.

Lorsque l'on effectue la condensation d'un dérivé.réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment diisopropyl éthyl amine, triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le N,N'-carbonyl-diimidazole ou l'éthoxy-2 éthoxycarbonyl-1 dihydro-1,2 quinoléine ou bien en milieu hydroorganique, en présence d'un agent alcalin de condensation comme le bicarbonate de sodium.

Selon l'invention les dérivés de perhydroisoindole pour lesquels R₁ est un radical phényle substitué par un radical hydroxy, peuvent être également obtenus par transformation d'un dérivé de perhydroisoindole pour lequel R₁ est un radical phényle substitué par un radical benzyloxy, par toute méthode connue qui n'affecte pas le reste de la molécule.

On opère par toute méthode connue pour l'élimination d'un radical benzyle, par exemple par hydrogénation catalytique en présence d'un catalyseur tel que l'hydroxyde de palladium à une température comprise entre 40 et 80°C, dans un solvant organique tel qu'un alcool par exemple. Eventuellement il est possible d'opérer sous pression (1 à 30 atmosphères).

Selon l'invention les dérivés de perhydroisoindole pour lesquels R₁ est un radical phényle substitué par aminopeuvent également être obtenus par transformation d'un dérivé de perhydroisoindole selon la revendication 1 pour lequel R₁ est un radical phényle substitué par un radical nitro par toute méthode de réduction connue qui n'affecte pas le reste de la molécule.

La réduction s'effectue notamment en présence de zinc en milieu hydroalcoolique.

Les acides de formule générale (II) peuvent être préparés selon les méthodes décrites ci-après dans les exemples, selon les méthodes décrites dans la demande de brevet EP 429 366 ou par analogie avec ces méthodes.

Le dérivé de perhydroisoindole de formule générale (III) peut être obtenu par action d'un composé organométallique de formule générale :

R₃-M (IV)

dans laquelle R₃ est défini comme précédemment, et M représente le lithium, ou un radical MgX ou CeX₂ pour lequel X est un atome d'halogène, sur le dérivé correspondant de perhydroisoindolone de formule générale : 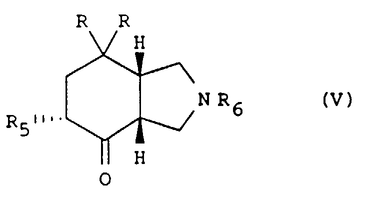 dans laquelle R est défini comme précédemment, R₅ est un radical hydroxy éventuellement protégé et R₆ est un radical protecteur, puis libération du radical protecteur de R₅ et élimination du radical protecteur R₆.

Le radical protecteur R₆ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle.

La réaction s'effectue en milieu anhydre, dans les conditions habituelles de réaction des composés organométalliques sur une cétone, qui n'affectent pas le reste de la molécule. Notamment on opère dans un éther (par exemple le tétrahydrofuranne ou l'éther éthylique) éventuellement en présence de chlorure de cérium anhydre à une température comprise entre -78 et 30°C. Il est entendu que selon la nature du radical protecteur du radical R₅, ce dernier peut être éliminé simultanément à la réaction.

L'élimination subséquente du radical protecteur R₆ s'effectue selon les méthodes habituelles. Notamment selon les méthodes décrites par T.W. Greene, par A. Wiley ou par Mc Omie dans les références citées précédemment.

Le dérivé de la perhydroisoindolone de formule générale (V) pour lequel R₅ est un radical hydroxy préalablement protégé peut être préparé par analogie avec la méthode décrite dans la demande de brevet européen EP 429 366, ou comme décrit ci-après dans les exemples.

Il est entendu que les dérivés de perhydroisoindole de formule générale (I), (III) et (V) peuvent présenter plusieurs formes stéréoisomères. Pour obtenir un produit de formule générale (I) de forme (3aR,7aR), la séparation des formes isomères est mise en oeuvre de préférence au niveau du dérivé de formule générale (III) ou au niveau d'un autre intermédiaire portant un radical oxo en position -4. La séparation s'effectue selon toute méthode connue et compatible avec la molécule.

A titre d'exemple, la séparation peut être effectuée par préparation d'un sel optiquement actif, par action de l'acide L(+) ou D(-) mandélique, de l'acide ditoluoyltartrique ou de l'acide dibenzoyltartrique, puis séparation des isomères par cristallisation. L'isomère recherché est libéré de son sel en milieu basique.

Les dérivés de l'isoindole de formule générale (I) peuvent être purifiés le cas échéant par des méthodes physiques telles que la cristallisation ou la chromatographie.

Le cas échéant, les dérivés de formule générale (I) pour lesquels les symboles R₁ et/ou R₂ contiennent des substituants amino ou alcoylamino, et les dérivés de formule générale (III) peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les dérivés de l'isoindole de formule générale (I) peuvent aussi, le cas échéant, lorsque R₂ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, N,N-diméthyléthanolamine, benzylamine, dicyclohexylamine, N-benzyl-β-phénéthylamine, N,N'-dibenzyléthylènediamine, diphénylènediamine, benzhydrylamine, quinine, choline, arginine, lysine, \leucine, dibenzylamine).

Les dérivés de l'isoindole de formule générale (III) ainsi que leurs sels sont des produits nouveaux qui sont également doués d'activité antagoniste de effets de la neurokinine A.

La neurokinine A est connue pour être impliquée dans un certain nombre de domaines pathologiques telles que l'asthme, la transmission de la douleur, les douleurs céphaliques, la migraine, les phénomènes inflammatoires, l'arthrite, les désordres mentaux neurodégénératifs, les désordres neurologiques, les désordres tensionnels, les désordres vésicaux, les cystites, les manifestations spasmodiques douloureuses et hypersécrétoires des voies digestives. [C.A. Maggi et coll., Drugs of the Future; 18(2), 155-158 (1993) ; C.A. Maggi et coll., J. Auton. Pharmacol., 13, 23-93 (1993)].

Les dérivés de l'isoindole selon la présente invention ainsi que leurs formes racémiques (3aRS,4RS,5RS,7aRS) et les dérivés de l'isoindole de formule générale (III), peuvent trouver une application dans les domaines où intervient la neurokinine A.

En effet, les produits selon l'invention manifestent une affinité pour les récepteurs à neurokinine A à des concentrations comprises entre 10 et 1000 nM (CI₅₀) mises en évidence dans la technique suivante :

Evaluation de l'affinité pour le récepteur NK₂ humain :

L'affinité des produits pour le récepteur NK₂ humain a été évaluée sur un homogénat lavé de cellules d'insecte (Spodoptera Frugiperda, SF₂₁) exprimant le récepteur NK₂ cloné à partir du jéjunum humain. L'expression de ce récepteur dans la lignée SF₂₁ est obtenue par infection des cellules à l'aide d'un baculovirus recombinant (BVE-hNK₂) possédant le gène du récepteur NK₂ étudié. L'affinité des produits a été mesurée en étudiant l'inhibition éventuelle de la fixation spécifique de la neurokinine A marquée à l'iode 125 (¹²⁵I-iodo-histidyl NKA) sur ces homogenats de cellule par différentes concentrations de produit. La fixation de la NKA iodée, en l'absence ou en présence de produit à évaluer, est mesurée par comptage de la radioactivité sur un compteur gamma après une incubation de 60 minutes à 25°C en présence de 0,1 nM de ligand radioactif et une filtration rapide sous pression réduite du milieu d'incubation. La fixation non spécifique est définie en présence de 5 µM de NKA non radioactive. La concentration de produit qui inhibe de 50% la fixation spécifique du ligand (CI₅₀) est déterminée par régression non linéaire en utilisant le programme de calcul de G.A. McPherson, Analysis of radioligand binding experiments, A collection of computer programs for the IBM PC. J. Pharmacol., Neth., 14, 213-228 (1985).

L'activité antagoniste, in vitro, des produits est déterminée vis à vis des contractions de l'artère pulmonaire de lapin induites par la [Lys⁵MeLeu⁹Nle¹⁰]NKA(4-10) selon la technique décrite par D. Regoli et coll., Trends Pharmacol. Sci, 9, 290-295 (1988) et P. D'Orléans-Juste et coll., Eur. J. Pharmacol., 125, 37-44 (1985). L'artère pulmonaire de lapin (mâle, albinos, néo-zélandais) est prélevée ; l'endothélium est détruit par introduction d'une baguette de verre enrobée de papier filtre dans le lumen de l'artère puis l'artère est découpée en hélice et mise à incuber dans une cuve à organes isolés contenant une solution de Krebs oxygénée (O₂ 95 % ; CO₂ 5 %) à 37°C sous une tension de 1 g. Après avoir déterminé l'activité agoniste de la [Lys⁵MeLeu⁹Nle¹⁰]NKA(4-10) (effet contractile), le produit à étudier est incubé pendant 2 heures dans la cuve à organes isolés avant une nouvelle addition de [Lys⁵MeLeu⁹Nle¹⁰]NKA(4-10). Le pourcentage d'inhibition obtenu est calculé. L'activité antagoniste d'un produit est exprimée en termes de CI₅₀.

Dans la technique ci-dessus, les produits étudiés manifestent un antagonisme des contractions de l'artère pulmonaire de lapin à la concentration suivante :

| | |
|---|---|
| Produit étudié | CI₅₀ (nM) |
| Exemple 1 | 215 |

Enfin, les dérivés de l'isoindole selon la présente invention ne présentent pas de toxicité, ils se sont montrés atoxiques chez la souris par voie sous cutanée à la dose de 40 mg/kg par voie sous-cutanée.

Les dérivés de l'isoindole selon l'invention, répondant à la formule générale (I) ou (III), sont également très intéressants par la synergie qu'ils procurent lorsqu'ils sont associés à des produits doués d'une activité antagoniste des récepteurs NK1.

Des antagonistes des récepteurs NK1 (antagonistes des effets de la substance P) sont connus et décrits notamment dans les demandes de brevet EP 429 366, EP 514 273, EP 514 275, WO 90/05525, WO 90/05729, WO 91/18899, WO 91/09844, WO 92/01688, WO 92/06079, WO 92/15585, WO 92/12151, WO 92/20661, WO 92/20676, WO 92/21677, WO 93/00330, WO 93/00331, WO 93/01159, WO 93/01169, WO 93/01165, WO 93/01170, WO 93/06099, WO 93/09116, WO 93/10073, WO 93/18023, WO 93/19064, WO 93/21155, WO 93/21181, WO 93/23380, EP 499 313, EP 394 989, EP 443 132, EP 482 539, EP 512 902, EP 517 589, EP 520 555, EP 522 808, EP 528 495, EP 532 456, EP 533 280, EP 536 817, EP 545 478, EP 559 538, XIIè Int. Symp. on Med. Chem., Bâle, 13-17 septembre 1992 ou 3rd Meeting of the European Neuropeptide Club (Cambridge 5-7 avril 1993). De tels produits peuvent être associés aux produits selon l'invention et permettent ainsi d'obtenir une potentialisation de effets antagonistes des récepteurs NK1 et NK2.

Les exemples suivants donnés à titre non limitatif illustrent la présente invention.

### Exemple 1 RPR106145

A une solution de 0,5 g de diphényl-7,7 (méthoxy-2 phényl)-4 perhydroisoindolediol-4,5-(3aR,4R,5R,7aR) et de 0,23 g d'acide indolyl-3 acétique dans 50 cm³ de dichlorométhane, refroidie à 0°C, on ajoute 5 mg d'hydrate d'hydroxy-1-benzotriazole, 0,28 g de 1-(3-diméthylaminopropyl)-3-éthylcarbodiimide et 0,24 cm³ de diisopropyléthylamine. On agite 2 heures à 0°C et 1 heure à température ambiante, ajoute 25 cm³ d'eau. La phase organique est décantée, séchée sur sulfate de magnésium et concentrée à sec sous pression réduite (2,7 kPa). Les cristaux jaunes obtenus sont chromatographiés sur colonne de gel de silice Merck (granulométrie 0,04-0,06 mm, diamètre 1,5 cm, hauteur 30 cm), en éluant sous une pression d'azote de 50 kPa, avec de l'acétate d'éthyle et en recueillant des fractions de 25 cm³. Les fractions 4 à 8 sont réunies puis concentrées à sec sous pression réduite (2,7 kPa). Le solide obtenu est cristallisé dans 5 cm³ d'oxyde d'isopropyle. On obtient 0,58 g de diphényl-7,7 (méthoxy-2 phényl)-4 [(indolyl-3) acétyl]-2 perhydroisoindolediol-4,5-(3aR,4R,5R,7aR) fondant à 218-220°C.

Le diphényl-7,7 (méthoxy-2 phényl)-4 perhydroisoindolediol-4,5- (3aR,4R,5R,7aR) peut être obtenu de la manière suivante :

A 2,3 g de ditoluoyl-1,4-D-tartrate de diphényl-7,7 (méthoxy-2 phényl)-4 perhydroisoindolediol-4,5-(3aR,4R,5R,7aR) en solution dans 46 cm³ de méthanol, on ajoute 100 cm³ d'eau et 6 cm³ de soude aqueuse 1N ; le mélange réactionnel est agité à température ambiante jusqu'à disparition du produit de départ. Les cristaux formés sont filtrés, essorés puis cristallisés dans 20 cm³ d'oxyde d'isopropyle. On obtient 1,4 g de diphényl-7,7 (méthoxy-2 phényl)-4 perhydroisoindolediol-4,5-(3aR,4R,5R,7aR) fondant à 183-185°C.

Le ditoluoyl-1,4-D-tartrate de diphényl-7,7 (méthoxy-2 phényl)-4 perhydroisoindolediol-4,5-(3aR,4R,5R,7aR) peut être préparé de la manière suivante:

A une solution de 7,5 g de diphényl-7,7 (méthoxy-2 phényl)-4 perhydroisoindolediol-4,5-(3aRS,4RS,5RS,7aRS) dans 75 cm³ de méthanol, on ajoute, sous agitation, 7,3 g d'acide (+) ditoluoyl-1,4-D-tartrique. Après dissolution totale, le mélange réactionnel est concentré à sec sous pression réduite (2,7 kPa); la meringue obtenue est recristallisée une première fois dans 100 cm³ d'acétonitrile puis une seconde fois dans 400 cm³ d'acétonitrile. Les cristaux obtenus sont recristallisés à pouvoir rotatoire constant dans un mélange d'éthanol et d'eau (60/40 en volumes). On obtient 2,3 g de ditoluoyl-1,4-D-tartrate de diphényl-7,7 (méthoxy-2 phényl)-4 perhydroisoindolediol-4,5- (3aR,4R,5R,7aR) fondant à 240°C.

Le diphényl-7,7 (méthoxy-2 phényl)-4 perhydroisoindolediol-4,5- (3aRS,4RS,5RS,7aRS) peut être préparé de la manière suivante :

Un mélange de 2 g de benzyl-2 diphényl-7,7 (méthoxy-2 phényl)-4 perhydroisoindolediol-4,5(3aRS,4RS,5RS,7aRS) et de 50 cm³ d'éthanol est chauffé à 65°C sous agitation; on ajoute 0,65 g d'hydroxyde de palladium sur charbon à 20% puis le mélange réactionnel est hydrogéné, sous agitation, à une température de 65°C et sous pression atmosphérique. Après 1 heure de réaction, le volume théorique d'hydrogène a été absorbé; le mélange réactionnel est filtré, puis concentré à sec sous pression réduite (2,7 kPa). Le résidu est cristallisé dans 10 cm³ d'oxyde d'isopropyle. On obtient 1,45 g de diphényl-7,7 (méthoxy-2 phényl) -4 perhydroisoindolediol-4,5-(3aRS, 4RS, 5RS, 7aRS) fondant à 230°C.

Le benzyl-2 diphényl-7,7 (méthoxy-2 phényl)-4 perhydroisoindolediol-4,5-(3aRS,4RS,5RS,7aRS) peut être préparé de la manière suivante :

A une suspension de 84,4 g de bromure de méthoxy-2 phénylmagnésium dans 1000 cm³ de tétrahydrofurane, on ajoute goutte à goutte, à température ambiante, sous agitation, une solution de 22 g d'acétoxy-5 benzyl-2 diphényl-7,7 perhydroisoindolone-4-(3aRS, 5RS, 7aRS) dans 220 cm³ de tétrahydrofurane. Le mélange réactionnel est agité à température ambiante pendant 18 heures, traité par 200 cm³ d'une solution aqueuse saturée en chlorure d'ammonium, repris par 200 cm³ d'oxyde d'éthyle et 200 g de glace. La phase organique est décantée, séchée sur sulfate de magnésium, filtrée et concentrée à sec sous pression réduite (2,7 kPa). Le résidu est cristallisé dans 250 cm³ d'éther de pétrole puis recristallisé dans 200 cm³ de méthanol; les cristaux sont lavés par 200 cm³ d'oxyde d'isopropyle. On obtient 16,4 g de benzyl-2 diphényl-7,7 (méthoxy-2 phényl)-4 perhydroisoindolediol-4,5-(3aRS,4RS,5RS,7aRS) fondant à 236°C.

L'acétoxy-5 benzyl-2 diphényl-7,7 perhydroisoindolone-4-(3aRS, 5RS, 7aRS) peut être préparée de la manière suivante :

A une solution de 86 g d'acétoxy-6 diphényl-4,4 cyclohexènone-2 et de 96 cm³ de N-butoxyméthyl N-triméthylsilylméthyl benzylamine dans 1000 cm³ de dichlorométhane, on ajoute 15 gouttes d'acide trifluoroacétique. Le mélange réactionnel est agité à température ambiante 15 heures puis on ajoute 2 g de carbonate de sodium et concentre à sec sous pression réduite (2,7 kPa). Le résidu est chromatographié sur colonne de gel de silice Merck (granulométrie 0,04-0,06 mm, diamétre 7 cm, hauteur 70 cm), en éluant sous une pression d'azote de 50 kPa, par un mélange de cyclohexane et d'acétate d'éthyle (20/80 en volumes) et en recueillant des fractions de 200 cm³. Les fractions 6 à 10 sont réunies et concentrées à sec sous pression réduite (2,7 kPa). On obtient 70 g d'acétoxy-5 benzyl-2 diphényl-7,7 perhydroisoindolone-4-(3aRS,5RS,7aRS) sous forme de miel (point de fusion inférieur à 40°C).

La N-butoxyméthyl N-triméthylsilylméthyl benzylamine peut être préparée selon la méthode de Y. TERAO et coll., Chem. Pharm. Bull., 33, 2762 (1985).

L'acétoxy-6 diphényl-4,4 cyclohexènone-2 peut être préparée selon la méthode décrite par W. OPPOLZER et coll., Helv. Chim. Acta, 59, 2012 (1976).

### Exemple 2 RPR102862

A une solution de 0,1 g de diphényl-7,7 (méthoxy-2 phényl)-4 perhydroisoindolediol-4,5-(3aR,4R,5R,7aR) et de 0,05 g d'acide (méthoxy-2 phényl)-2 propanoïque-(S) dans 5 cm³ de dichlorométhane, refroidie à 0°C, on ajoute 5 mg d'hydrate d'hydroxy-1-benzotriazole, 0,06 g de 1-(3-diméthylaminopropyl)-3-éthylcarbodiimide et 0,04 cm³ de diisopropyléthylamine. On agite 4 heures à température ambiante, ajoute 30 cm³ d'eau, 10 cm³ d'une solution aqueuse saturée de bicarbonate de sodium et 50 cm³ d'une solution aqueuse saturée de chlorure de sodium. La phase organique est décantée, séchée sur sulfate de magnésium et concentrée à sec sous pression réduite (2,7 kPa). Le résidu jaune obtenu est chromatographié sur colonne de gel de silice Merck (granulométrie 0,04-0,06 mm, diamètre 1,5 cm, hauteur 30 cm), en éluant sous une pression d'azote de 50 kPa, par un mélange de cyclohexane et d'acétate d'éthyle (60/40 en volumes) et en recueillant des fractions de 10 cm³. Les fractions 8 à 13 sont réunies puis concentrées à sec sous pression réduite (2,7 kPa). On obtient 0,08 g de diphényl-7,7 (méthoxy-2 phényl)-4 [(méthoxy-2 phényl)-2 propionyl-(S)]-2 perhydroisoindolediol-4,5-(3aR,4R,5R,7aR) fondant à 172°C.

L'acide (méthoxy-2 phényl)-2 propionique-(S) peut être préparé, par analogie avec les méthodes décrites par D.A. Evans et coll., Tetrahedron, 44, 5525, (1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D}²⁰ = +84,6° (c=1; CHCl₃).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2-(4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80% dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle; après décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

### Exemple 3 RPR107539

En opérant selon le mode opératoire de l'exemple 1, à partir de 0,2 g de diphényl-7,7 (méthoxy-2 phényl)-4 perhydroisoindolediol-4,5-(3aR,4R,5R,7aR) et de 0,11 g d'acide (méthoxy-2 phényl)-2 propionique-(R), on obtient 0,12 g de diphényl-7,7 (méthoxy-2 phényl)-4 [(méthoxy-2 phényl) -2 propionyl-(R)]-2 perhydroisoindolediol-4,5-(3aR,4R,5R,7aR) fondant à 178-180°C.

L'acide (méthoxy-2 phényl)-2 propionique-(R) peut être préparé, par analogie avec les méthodes décrites par D.A Evans et coll., Tetrahedron, 44, 5525,(1988), selon le mode opératoire suivant:

A une solution refroidie à +5°C de 10,88 g de méthyl-4 phényl-5 [(méthoxy-2 phényl)-2-(R)-propionyl]-3 oxazolidinone-2-(4R,5R) dans 300 cm³ de tétrahydrofuranne et 100 cm³ d'eau, on ajoute 2,71 g d'hydroxyde de lithium et 13 cm³ de solution aqueuse à 30% de peroxyde d'hydrogène. Le mélange réactionnel est agité 3 heures à cette tempétature, puis, après retour à la température ambiante, on ajoute une solution aqueuse de sulfite de sodium, puis du dichlorométhane et on décante. La phase aqueuse est acidifiée par une solution aqueuse d'acide chlorhydrique 4N, extraite par du dichlorométhane, puis la phase organique est séchée sur sulfate de magnésium, et concentrée à sec sous pression réduite (2,7 kPa). Le solide obtenu est recristallisé dans l'hexane, essoré et séché. On obtient 5,24 g d'acide (méthoxy-2 phényl)-2 propionique-(R) sous la forme de cristaux blancs fondant à 102°C ; [α]_{D}²⁰ = - 76° ( c=1 ; méthanol).

La méthyl-4 phényl-5 [(méthoxy-2 phényl)-2-(R)-propionyl]-3 oxazolidinone-2-(4R,5R) peut être préparée de la manière suivante :

A une solution refroidie à -50°C de 22,82 g de méthyl-4 phényl-5 [(méthoxy-2 phényl)-acétyl]-3 oxazolidinone-2-(4R,5R) dans 190 cm³ de tétrahydrofuranne on ajoute 19,31 g d'hexaméthyl-1,1,1,3,3,3 disilazanate de sodium, on agite 45 minutes à cette température, puis on ajoute 8,82 cm³ d'iodure de méthyle. Le mélange réactionnel est ensuite agité 15 heures à température ambiante, puis on ajoute 18,65 cm³ d'acide acétique, on dilue par 200 cm³ d'acétate d'éthyle, lave par 200 cm³ d'eau, puis par 250 cm³ d'une solution aqueuse saturée de chlorure de sodium, séche sur sulfate de magnésium et concentre à sec sous pression réduite (2,7 kPa). Le résidu obtenu est cristallisé dans l'oxyde d'isopropyle, essoré et séché. On obtient 10,95 g de méthyl-4 phényl-5 [(méthoxy-2 phényl)-2-(R)-propionyl]-3 oxazolidinone-2-(4R,5R) sous la forme d'un solide blanc fondant à 110°C.

La méthyl-4 phényl-5 (méthoxy-2 phénylacétyl)-3 oxazolidinone-2-(4R,5R) peut être préparée de la manière suivante :

A une solution de 27,4 g de méthyl-4 phényl-5 oxazolidinone-2-(4R,5R) dans 200 cm³ de tétrahydrofuranne sec on ajoute à -78°C, 100 cm³ d'une solution de butyllithium 1,6 M dans l'hexane, puis on ajoute une solution de 33,12 g de chlorure d'acide méthoxy-2 phénylacétique dans 30 cm³ de tétrahydrofurane. On agite 30 minutes à cette température puis on laisse revenir à température ambiante. On verse ensuite 200 cm³ d'une solution aqueuse saturée de chlorure d'ammonium, puis 200 cm³ d'acétate d'éthyle. La phase organique est lavée trois fois par 300 cm³ d'eau, puis par 300 cm³ d'une solution aqueuse saturée en chlorure de sodium, séchée sur sulfate de magnésium et concentrée à sec sous pression réduite (2,7 kPa). Le résidu est repris par 100 cm³ de diéthyle oxyde. On obtient 35,5 g de méthyl-4 phényl-5 (méthoxy-2 phénylacétyl)-3 oxazolidinone-2-(4R,5R) sous la forme de cristaux blancs fondant à 126°C.

### Exemple 4 RPR107587

En opérant selon le mode opératoire de l'exemple 1, à partir de 2 g de diphényl-7,7 (méthoxy-2 phényl)-4 perhydroisoindolediol-4,5-(3aR,4R,5R,7aR) et de 1,3 g d'acide (benzyloxy-2) phényl acétique, après purification par chromatographie sur colonne de gel de silice Merck (granulométrie 0,04-0,06 mm, diamètre 2 cm, hauteur 28 cm), et en éluant sous une pression de 0,5 bar par un mélange de dichlorométhane et de méthanol (97,5/2,5 en volumes), on obtient 1,1 g de diphényl-7,7 (méthoxy-2 phényl)-4 [(benzyloxy-2 phényl) acétyl]-2 perhydroisoindolediol-4,5-(3aR,4R,5R,7aR) fondant à 176°C.

### Exemple 5 RPR107588

Un mélange de 1,5 g de diphényl-7,7 (méthoxy-2 phényl)-4 [(benzyloxy-2 phényl)acétyl]-2 perhydroisoindolediol-4,5-(3aR,4R,5R,7aR) et de 50 cm³ d'éthanol est chauffé à 60°C sous agitation; on ajoute 0,5 g d'hydroxyde de palladium sur charbon à 20% puis le mélange réactionnel est hydrogéné, sous agitation, à une température de 60°C et sous pression atmosphérique. Après 45 minutes de réaction, le volume théorique d'hydrogène a été absorbé; le mélange réactionnel est filtré, puis concentré à sec sous pression réduite (2,7 kPa). La meringue obtenue est purifiée par cristallisation dans 10 cm³ d'oxyde d'isopropyle pour donner 1,09 g de diphényl-7,7 (méthoxy-2 phényl)-4 [(hydroxy-2 phényl) acétyl]-2 perhydroisoindolediol-4,5-(3aR,4R,5R, 7aR) fondant à 188°C.

### Exemple 6 RPR108208

En opérant comme à l'exemple 1, à partir de 0,62 g de diphényl-7,7 (méthoxy-2 phényl)-4 perhydroisoindolediol-4,5- (3aR,4R, 5R,7aR) et de 0,30 g d'acide (diméthylamino-2) phényl acétique, on obtient 0,35 g de diphényl-7,7 (méthoxy-2 phényl)-4 [(diméthylamino-2 phényl) acétyl]-2 perhydroisoindolediol-4,5-(3aR,4R,5R,7aR) fondant à 175-177°C.

### Exemple 7 RPR110717

En opérant selon le mode opératoire de l'exemple 1 à partir de 1,25 g de diphényl-7,7 (méthoxy-2 phényl)-4 perhydroisoindolediol-4,5-(3aR,4R,5R,7aR) et de 0,95 g d'acide [(diméthylamino-2 éthyl)-1 indolyl-3] acétique, on obtient 1,4 g de diphényl-7,7 (méthoxy-2 phényl)-4 [((diméthylamino-2 éthyl)-1 indolyl-3) acétyl]-2 perhydroisoindolediol-4,5-(3aR,4R,5R,7aR) fondant à 210-212°C.

L'acide [(diméthylamino-2 éthyl)-1 indolyl-3] acétique peut être préparé selon la méthode de ANDREANI et coll., Acta Pharm. Nord. 1991, 3(3), 125.

### Exemple 8 RPR111271

En opérant selon le mode opératoire de l'exemple 1 à partir de 0,5 g de diphényl-7,7 (méthoxy-2 phényl)-4 perhydroisoindolediol-4,5- (3aR, 4R,5R,7aR) et de 0,26 g d'acide (hydroxy-5 indolyl)-3 acétique, on obtient 0,20 g de diphényl-7,7 (méthoxy-2 phényl)-4 [(hydroxy-5 indolyl-3) acétyl]-2 perhydroisoindolediol-4,5-(3aR,4R,5R,7aR) fondant à 230-235°C.

Les exemples suivants illustrent la préparation des formes racémiques des dérivés de l'isoindole de formule générale (I). En opérant par analogie avec ces exemples, les dérivés de perhydroisoindole de forme (3aR,4R,5R,7aR) correspondants peuvent être préparés.

### Exemple A RPR106891

A une solution de 0,42 g de diphényl-7,7 (méthoxy-2 phényl)-4 perhydroisoindolediol-4,5-(3aRs,4Rs,5Rs,7aRs) dans 10 cm³ de dichlorométhane, on ajoute 0,21 cm³ de diisopropyléthylamine puis 0,23 g du chlorure de l'acide naphtyl-2 acétique dans 5,5 cm³ de dichlorométhane. On agite à température ambiante pendant 1 heure, ajoute 25 cm³ d'eau. La phase organique est décantée, séchée sur sulfate de magnésium et concentrée à sec sous pression réduite (2,7 kPa). Le résidu est repris par 50 cm³ d'acétate d'éthyle, 3 cm³ d'acide chlorhydrique aqueux 1N et 40 cm³ d'eau. La phase organique est décantée, séchée sur sulfate de magnésium et concentrée à sec sous pression réduite (2,7 kPa). Le solide obtenu est cristallisé une première fois dans 5 cm³ de propanol-2 puis une seconde fois dans 5 cm³ d'oxyde d'isopropyle. On obtient 0,3 g de diphényl-7,7 (méthoxy-2 phényl)-4 [(naphtyl-2) acétyl]-2 perhydroisoindolediol-4,5- (3aRs,4Rs,5Rs,7aRs) fondant à 188-190°C.

Le chlorure de l'acide naphtyl-2 acétique est obtenu à partir d'un mélange de 0,38 g d'acide naphtyl-2 acétique et de 4 cm³ de chlorure de thionyle, porté à reflux 20 minutes. Après concentration à sec sous pression réduite (2,7 kPa), on obtient 0,41 g d'une huile utilisée à l'état brut dans les synthèses ultérieures.

### Exemple B RPR106967

En opérant selon le mode opératoire de l'exemple A, à partir de 0,62 g de diphényl-7,7 (méthoxy-2 phényl)-4 perhydroisoindolediol-4,5-(3aRS,4RS,5RS,7aRS) et de 0,35 g du chlorure de l'acide naphtyl-1 acétique, on obtient 0,4 g de diphényl-7,7 (méthoxy-2 phényl)-4 [(naphtyl-1) acétyl]-2 perhydroisoindolediol-4,5- (3aRs,4Rs,5Rs,7aRs) fondant à 188-190°C.

### Exemple C RPR106966

En opérant selon le mode opératoire de l'exemple 1, à partir de 0,83 g de diphényl-7,7 (méthoxy-2 phényl)-4 perhydroisoindolediol-4,5-(3aRS,4RS,5RS,7aRS) et de 0,43 g d'acide (fluoro-5 indolyl)-3 acétique, on obtient 0,8 g de diphényl-7,7 (méthoxy-2 phényl)-4 [(fluoro-5 indolyl-3) acétyl]-2 perhydroisoindolediol-4,5- (3aRS,4RS, 5RS,7aRS) fondant à 248°C.

### Exemple D RPR107091

En opérant selon le mode opératoire de l'exemple 1, à partir de 0,62 g de diphényl-7,7 (méthoxy-2 phényl)-4 perhydroisoindolediol-4,5-(3aRS, 4RS,5RS,7aRS) et de 0,34 g d'acide (méthoxy-5 indolyl)-3 acétique, on obtient 0,78 g de diphényl-7,7 (méthoxy-2 phényl)-4 [(méthoxy-5 indolyl-3) acétyl]-2 perhydroisoindolediol-4,5- (3aRs, 4RS,5RS,7aRS) fondant à 204-206°C.

### Exemple E RP 100949

A une solution de 4,5 g de chlorhydrate de diphényl-7,7 (méthoxy-2 phényl)-4 perhydroisoindolediol-4,5- (3aRs,4Rs,5Rs,7aRs) dans 100 cm³ de dichlorométhane, refroidie à 0°C, on ajoute 4,2 cm³ de triéthylamine puis 2,4 g du chlorure de l'acide (méthoxy-2 phényl)acétique dans 50 cm³ de dichlorométhane. On agite à température ambiante pendant 90 minutes; le mélange réactionnel est lavé par 2 fois 10 cm³ d'eau, séché sur sulfate de magnésium et concentré à sec sous pression réduite (2,7 kPa). Le solide cristallisé est repris par 100 cm³ d'oxyde de diisopropyle puis filtré, lavé par 50 cm³ d'une solution saturée de bicarbonate de sodium puis 50 cm³ d'oxyde de diisopropyle. On obtient 4,35 g de diphényl-7,7 (méthoxy-2 phényl)-4 (méthoxy-2 phényl) acétyl-2 perhydroisoindolediol-4,5-(3aRs,4Rs,5Rs,7aRs) sous forme d'un solide beige clair fondant à 278°C.

Le chlorure de l'acide (méthoxy-2 phényl)acétique est obtenu à partir d'un mélange de 2,2 g d'acide (méthoxy-2 phényl)acétique et de 20 cm³ de chlorure de thionyle, porté à reflux 30 minutes. Après concentration à sec sous pression réduite (2,7 kPa), on obtient 2,4 g d'une huile jaune utilisée à l'état brut dans les synthèses ultérieures.

Le chlorhydrate de diphényl-7,7 (méthoxy-2 phényl)-4 perhydroisoindolediol-4,5 (3aRS,4RS,5RS,7aRS) peut être préparé de la manière suivante :

A une solution de 5,15 g de diphényl-7,7 (méthoxy-2 phényl)-4 tert-butoxycarbonyl-2 perhydroisoindolediol-4,5-(3aRS,4RS,5RS,7aRS) dans 25 cm³ de dioxanne, on ajoute à température ambiante une solution de 25 cm³ de dioxanne chlorhydrique 6N. Le mélange réactionnel est agité 1 heure à cette température, puis concentré à sec sous pression réduite (2,7 kPa). Le résidu est lavé par 20 cm³ l'acétonitrile, essoré et séché. On obtient 4,5 g de chlorhydrate de diphényl-7,7 (méthoxy-2 phényl)-4 perhydroisoindolediol-4,5-(3aRs,4Rs,5Rs,7aRs) sous forme de cristaux blancs fondant à une température supérieure à 300°C.

Le diphényl-7,7 (méthoxy-2 phényl)-4 tert-butoxycarbonyl-2 per-hydroisoindolediol-4,5-(3aRS,4RS,5RS,7aRS) peut être préparé de la manière suivante :

A une suspension de 26,4 g d'acétoxy-5 diphényl-7,7 (méthoxy-2 phényl)-4 tert-butoxycarbonyl-2 perhydroisoindolone-4-(3aRS,5RS,7aRs) et de 43,3 g de chlorure de cérium anhydre dans 265 cm³ de tétrahydrofuranne sec, on ajoute goutte à goutte à température ambiante sous agitation une suspension de 30,9 g de bromure de méthoxy-2 phénylmagnésium dans 170 cm³ de tétrahydrofuranne sec. Le mélange réactionnel est agité à température ambiante pendant 24 heures, traité par 400 cm³ d'une solution aqueuse saturée en chlorure d'ammonium, repris par 1000 cm³ d'acétate d'éthyle puis filtré sur célite. La phase organique est décantée, séchée sur sulfate de magnésium, filtrée et concentrée à sec sous pression réduite (2,7 kPa). Le résidu est chromatographié sur colonne de gel de silice Merck (granulométrie 0,04-0,06 mm, diamètre 7 cm, hauteur 55 cm), en éluant sous une pression d'azote de 50 kPa, par un mélange de cyclohexane et d'acétate d'éthyle (70/10 en volumes) et en recueillant des fractions de 250 cm³. Les fractions 10 à 19 sont réunies et concentrées à sec sous pression réduite (2,7 kPa). On obtient 18 g de diphényl-7,7 (méthoxy-2 phényl)-4 tert-butoxycarbonyl-2 perhydroisoindolediol-4,5-(3aRS,4RS,5RS,7aRS) sous forme de cristaux blancs fondant à 229°C.

L'acétoxy-5 diphényl-7,7 tert-butoxycarbonyl-2 perhydroisoindolone-4-(3aRS,5RS,7aRS) peut être préparé de la manière suivante :

A une suspension de 19 g de chlorhydrate d'acétoxy-5 diphényl-7,7 perhydroisoindolone-4-(3aRs,5Rs,7aRS) dans 200 cm³ de dichlorométhane sec, on ajoute à une température voisine de 5°C, sous agitation, 46,9 cm³ de triéthylamine, 11,8 g de di-tert-butyl dicarbonate, puis 0,3 g de diméthylamino-4 pyridine. Le mélange réactionnel est agité à température ambiante pendant 24 heures puis lavé par une solution aqueuse saturée en bicarbonate de sodium. La phase organique est décantée, séchée sur sulfate de magnésium, filtrée et concentrée à sec sous pression réduite (2,7 kPa). Le résidu est cristallisé dans 120 cm³ d'oxyde de diisopropyle. On obtient 21 g d'acétoxy-5 diphényl-7,7 perhydroisoindolone-4-(3aRS,5RS,7aRS) sous forme de cristaux blancs fondant à 213°C.

Le chlorhydrate d'acétoxy-5 diphényl-7,7 perhydroisoindolone-4-(3aRS,5RS,7aRS) peut être préparé de la manière suivante :

A une solution de 51,2 g d'acétoxy-5 diphényl-7,7 vinyloxycarbonyl-2 perhydroisoindolinone-4-(3aRS,5RS,7aRS) dans 118 cm³ de dioxanne, on ajoute à température ambiante, une solution de 394 cm³ de dioxanne chlorhydrique 5,2 N. Le mélange réactionnel est agité 1 heure à cette température, puis concentré à sec sous pression réduite (2,7 kPa). Le résidu est recristallisé dans 200 cm³ d'éthanol bouillant. On obtient 13,4 g de chlorhydrate d'acétoxy-5 diphényl-7,7 perhydroisoindolone-4-(3aRS,5RS,7aRS) sous forme de cristaux blancs fondant à une température supérieure à 300°C.

L'acétoxy-5 diphényl-7,7 vinyloxycarbonyl-2 perhydroisoindolinone-4-(3aRS,5RS,7aRS) peut être préparé de la manière suivante :

A une solution de 58 g d'acétoxy-5 benzyl-2 diphényl-7,7 perhydroisoindolinone-4-(3aRS,5RS,7aRS) dans 580 cm³ de dichlorométhane sec, on ajoute à température ambiante sous agitation 13,6 cm³ de chloroformiate de vinyle. Le mélange réactionnel est porté à reflux du solvant pendant une heure, refroidi à température ambiante et concentré à sec sous pression réduite (2,7 kPa). Le résidu est cristallisé dans 400 cm³ d'un mélange d'oxyde de diisopropyle et d'éther de pétrole (50/50 en volumes). On obtient 51,4 g d'acétoxy-5 diphényl-7,7 vinyloxycarbonyl-2 perhydroisoindolone-4-(3aRS,5RS,7aRS) sous forme de cristaux jaunes fondant à 205-210°C.

### Exemple F RPR 106965

En opérant selon le mode opératoire de l'exemple 1, à partir de 0,62 g de diphényl-7,7 (méthoxy-2 phényl)-4 perhydroisoindolediol-4,5-(3aRS,4RS,5RS,7aRS) et de 0,31 g d'acide (N-méthyl indolyl)-3 acétique, on obtient 0,55 g de diphényl-7,7 (méthoxy-2 phényl)-4 [(N-méthyl indolyl-3) acétyl]-2 perhydroisoindolediol-4,5-(3aRS,4RS, 5RS,7aRS) fondant à 240°C.

### Exemple G RPR107108

En opérant selon le mode opératoire de l'exemple 1, à partir de 0,62 g de diphényl-7,7 (méthoxy-2 phényl)-4 perhydroisoindolediol-4,5-(3aRS,4RS,5RS,7aRS) et de 0,30 g d'acide (diméhylamino-2) phényl acétique, on obtient 0,47 g de diphényl-7,7 (méthoxy-2 phényl)-4 [(diméthylamino-2 phényl) acétyl]-2 perhydroisoindolediol-4,5- (3aRs, 4RS,5RS,7aRS) fondant à 250°C.

### Exemple H RPR108329

En opérant selon le mode opératoire de l'exemple 1 à partir de 0,62 g de diphényl-7,7 (méthoxy-2 phényl)-4 perhydroisoindolediol-4,5-(3aRS,4RS,5RS,7aRS) et de 0,40 g d'acide (pyrrolidinyl-1)-2 phényl acétique, on obtient 0,70 g de diphényl-7,7 (méthoxy-2 phényl)-4 [((pyrrolidinyl-1)-2 phényl) acétyl]-2 perhydroisoindolediol-4,5-(3aRS,4RS,5RS,7aRS) fondant à 236°C.

### Exemple I RPR110166

A une solution de 1 g de diphényl-7,7 (méthoxy-2 phényl)-4 [(nitro-4 phényl) acétyl]-2 perhydroisoindolediol-4,5- (3aRs,4Rs,5Rs,7aRs) dans 3,3 cm³ de méthanol, maintenue à une température de 50°C, on ajoute 33 cm³ d'eau, 4,6 g de chlorure d'ammonium et 2,2 g de zinc en poudre. Le mélange réactionnel est porté au reflux 30 minutes, refroidi à température ambiante puis lavé par 2 fois 50 cm³ de dichlorométhane. La phase organique est décantée, séchée sur du sulfate de magnésium, filtrée et concentrée à sec sous pression réduite (2,7 kPa). Le résidu est repris par 70 cm³ d'acide chlorhydrique 1N. La phase organique est extraite par 2 fois 50 cm³ d'acétate d'éthyle. La phase aqueuse est alcalinisée par de la soude 1N puis la phase organique est extraite par 3 fois 100 cm³ d'acétate d'éthyle. Elle est décantée, séchée sur sulfate de magnésium et concentrée à sec sous pression réduite (2,7 kPa). La meringue obtenue est cristallisée dans 20 cm³ d'oxyde d'isopropyle. On obtient 0,71 g de diphényl-7,7 (méthoxy-2 phényl)-4 [(amino-4 phényl) acétyl]-2 perhydroisoindolediol-4,5-(3aRS,4Rs,5Rs,7aRs) fondant à 188°C.

Le diphényl-7,7 (méthoxy-2 phényl)-4 [(nitro-4 phényl) acétyl]-2 perhydroisoindolediol-4,5-(3aRS,4Rs,5Rs,7aRs) peut être préparé de la manière suivante:

En opérant selon le mode opératoire de l'exemple 1 à partir de 2 g de diphényl-7,7 (méthoxy-2 phényl)-4 perhydroisoindolediol-4,5-(3aRS, 4RS,5RS,7aRS) et de 0,92 g d'acide nitro-4 phényl acétique, on obtient 1,25 g de diphényl-7,7 (méthoxy-2 phényl)-4 [(nitro-4 phényl) acétyl]-2 perhydroisoindolediol-4,5-(3aRS,4RS,5RS,7aRS).

### Exemple J RPR110169

En opérant selon le mode opératoire de l'exemple 1 à partir de 1 g de diphényl-7,7 (méthoxy-2 phényl)-4 perhydroisoindolediol-4,5-(3aRS, 4RS,5RS,7aRS) et de 0,52 g d'acide (diméhylamino-4) phényl acétique, on obtient 1,03 g de diphényl-7,7 (méthoxy-2 phényl)-4 [(diméthylamino-4 phényl) acétyl]-2 perhydroisoindolediol-4,5-(3aRS, 4RS,5RS,7aRS) fondant à 189°C.

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, sublinguale, rectale, topique, oculaire, intranasale ou en aérosols à visée pulmonaire.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

Les compositions pour administration oculaires peuvent être des instillations.

Les compositions pour administration intranasale peuvent être des solutions ou des poudres pharmaceutiquement acceptables destinées à des gouttes ou à des pulvérisations.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, du sérum ou tout autre véhicule pharmaceutiquement acceptable. Pour l'usage sous forme d'aérosols secs destinés à être directement inhalés, le principe actif est est finement divisé et associé à un diluant ou véhicule solide hydrosoluble d'une granulométrie de 30 à 80 µm, par exemple le dextrane, le mannitol ou le lactose.

En thérapeutique humaine, les produits selon l'invention peuvent être particulièrement utiles dans les traitements des manifestations spasmodiques, douloureuses et inflammatoires des voies respiratoires (asthme, bronchites asthmatiformes, hypersécretion bronchique, bronchite aigüe et chronique, rhinites, toux, trachéites), des voies digestives (gastrites, gastralgies, diarrhées, colites ulcéreuses, syndrome du colon irritable, maladie de Crohn) et des voies urinaires (hyperréflexies urinaires, cystites). Ils sont également utiles dans le traitement des douleurs d'origine traumatique, post-chirurgicale, menstruelle, céphaliques, dans les algies vasculaires de la face (cluster headache) et dans les traitements des migraines. Les nouveaux dérivés de l'isoindole sont également utiles dans le traitement de l'inflammation en rhumatologie, dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses, les brûlures et dans les troubles inflammatoires dentaires ou oculaires et dans le domaine des sécretions lachrymales. Les produits selon l'invention peuvent également trouver une application dans les traitements de maladies neurologiques, maladie de Parkinson, maladie d'Alzeimer, dans les traitements des maladies inflammatoires et/ou autoimmunes et/ou démyélinisantes du système nerveux central et/ou périphérique (sclérose en plaque, syndrome de Guillain-Barré, encéphalopathies d'origine virale ...), dans les syndromes neurologiques en relation avec une extravasation plasmatique (oedème de la moelle épinière, oedème cérébral...), en relation avec une atteinte de la barrière hématoencéphalique ou dans tout syndrome neurologique spastique (traitements myorelaxants). Les produits selon l'invention peuvent également être utiles dans les traitements de l'anxiété, des psychoses, de la schizophrénie, ou encore dans les traitements des troubles cardiovasculaires tels de l'hypotension. Une autre application peut être également le traitement des troubles gynécologiques, le traitement des troubles liés à une mauvaise régulation de la croissance (nanisme, hypotrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### Exemple

On prépare, selon la technique habituelle, des comprimés de produit actif ayant la composition suivante :
- diphényl-7,7 (méthoxy-2 phényl)-4 [(indolyl-3) acétyl]-2 perhydroisoindolediol-4,5-(3aR,4R,5R,7aR) 25 mg
- amidon 83 mg
- silice 30 mg
- stéarate de magnésium 3 mg

## Revendications

1. Un dérivé de perhydroisoindole caractérisé en ce qu'il répond à la formule générale : 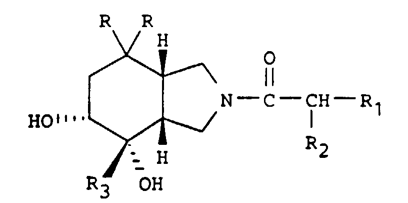 dans laquelle
- les symboles R sont identiques et représentent des radicaux phényle éventuellement substitués par un atome d'halogène ou par un radical méthyle en position 2 ou 3,
- le symbole R₁ représente un radical phényle éventuellement substitué par un ou plusieurs atomes d'halogène ou radicaux hydroxy, benzyloxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle, indényle ou hétérocyclyle mono ou polycyclique, saturé ou insaturé contenant 5 à 9 atomes de carbone et un ou plusieurs hétéroatomes choisis parmi l'oxygène, l'azote ou le soufre, et éventuellement substitué par un atome d'halogène ou par un radical alcoyle, alcoyloxy, aminoalcoyle, alcoylaminoalcoyle ou dialcoylaminoalcoyle,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino, et
- le symbole R₃ représente un radical phényle éventuellement substitué en position -2 par un radical alcoyle ou alcoyloxy contenant 1 ou 2 atomes de carbone,
les radicaux alcoyle et acyle cités ci-dessus étant (sauf mention spéciale) droits ou ramifiés et contenant 1 à 4 atomes de carbone, ainsi que ses sels lorsqu'ils existent.

2. Un dérivé de perhydroisoindole selon la revendication 1, caractérisé en ce que le symbole R₁ est un radical hétérocyclyle mono ou polycyclique, saturé ou insaturé choisi parmi thiényle, furyle, pyridyle, dithiinyle, indolyle, isoindolyle, benzothiényle, thiazolyle, isothiazolyle, oxazolyle, imidazolyle, pyrrolyle, triazolyle, thiadiazolyle, quinolyle, isoquinolyle, ou naphtyridinyle.

3. Un dérivé de perhydroisoindole selon la revendication 1, caractérisé en ce qu'il s'agit du diphényl-7,7 (méthoxy-2 phényl)-4 [((diméthylamino-2 éthyl)-1 indolyl-3) acétyl]-2 perhydroisoindolediol-4,5-(3aR,4R,5R,7aR).

4. Un dérivé de perhydroisoindole selon la revendication 1, caractérisé en ce qu'il s'agit du diphényl-7,7 (méthoxy-2 phényl)-4 [(hydroxy-2 phényl) acétyl]-2 perhydroisoindolediol-4,5-(3aR,4R,5R, 7aR).

5. Un dérivé de perhydroisoindole selon la revendication 1, caractérisé en ce qu'il s'agit du diphényl-7,7 (méthoxy-2 phényl)-4 [(benzyloxy-2 phényl) acétyl]-2 perhydroisoindolediol-4,5-(3aR,4R,5R, 7aR).

6. Un dérivé de perhydroisoindole selon la revendication 1, caractérisé en ce qu'il s'agit du diphényl-7,7 (méthoxy-2 phényl)-4 [(indolyl-3) acétyl]-2 perhydroisoindolediol-4,5-(3aR,4R,5R,7aR).

7. Un dérivé de perhydroisoindole selon la revendication 1, caractérisé en ce qu'il s'agit du diphényl-7,7 (méthoxy-2 phényl)-4 [(hydroxy-5 indolyl-3) acétyl]-2 perhydroisoindolediol-4,5-(3aR,4R, 5R,7aR).

8. Procédé de préparation d'un dérivé de perhydroisoindole selon la revendication 1, caractérisé en ce que l'on fait agir un acide ou un dérivé réactif de l'acide de formule générale : 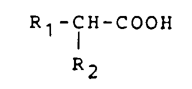 dans laquelle R₁ et R₂ sont définis comme dans la revendication 1, sur un dérivé de l'isoindole de formule générale : 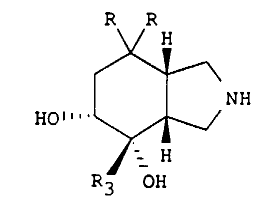 dans laquelle les symboles R et R₃ sont définis comme dans la revendication 1, puis transforme éventuellement le produit obtenu en un sel, lorsqu'ils existent.

9. Procédé de préparation d'un dérivé de perhydroisoindole selon la revendication 1 pour lequel R₁ est un radical phényle substitué par hydroxy, caractérisé en ce que l'on transforme un dérivé de perhydroisoindole selon la revendication 1 pour lequel R₁ est un radical phényle substitué par un radical benzyloxy par toute méthode connue qui n'affecte pas le reste de la molécule.

10. Procédé de préparation d'un dérivé de perhydroisoindole selon la revendication 1 pour lequel R₁ est un radical phényle substitué par amino, caractérisé en ce que l'on transforme un dérivé de perhydroisoindole selon la revendication 1 pour lequel R₁ est un radical phényle substitué par un radical nitro par toute méthode de réduction connue qui n'affecte pas le reste de la molécule.

11. Application des dérivés racémiques (3aRS,4RS,5RS,7aRS) correspondants aux dérivés de perhydroisoindole définis dans la revendication 1, à la préparation d'un médicament destiné aux traitements des affections médiées par le récépteur à tachykinines NK2.

12. Un dérivé de perhydroisoindole caractérisé en ce qu'il répond à la formule générale : 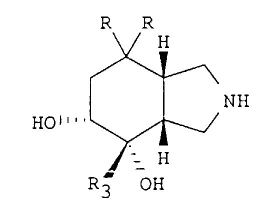 dans laquelle R et R₃ sont définis comme dans la revendication 1, ainsi que ses sels.

13. Associations synergisantes des effets antagonistes des récepteurs NK1 et NK2, constituées d'un dérivé de perhydroisoindole selon l'une des revendications 1 ou 7 et d'un produit connu pour son activité antagoniste des récepteurs NK1.

14. Composition pharmaceutique caractérisée en ce qu'elle contient au moins un produit selon la revendication 1, à l'état pur ou sous forme d'une association avec un ou plusieurs adjuvants ou diluants compatibles et pharmaceutiquement acceptables.

## Patentansprüche

1. Perhydroisoindol-Derivat, dadurch gekennzeichnet, daß es der allgemeinen Formel 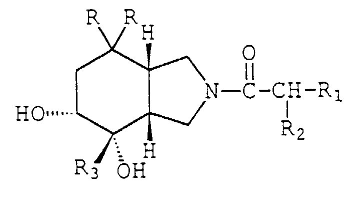 entspricht, in der
- die Symbole R gleich sind und Phenylreste darstellen, gegebenenfalls substituiert durch ein Halogenatom oder einen Methylrest in Position 2 oder 3,
- das Symbol R₁ einen Phenylrest darstellt, gegebenenfalls substituiert durch ein oder mehrere Halogenatome oder Reste Hydroxy, Benzyloxy, Alkyl, das gegebenenfalls substituiert sein kann (durch Halogenatome oder Reste Amino, Alkylamino oder Dialkylamino), Alkyloxy oder Alkylthio, das gegebenenfalls substituiert sein kann [durch Reste Hydroxy, Amino, Alkylamino oder Dialkylamino, gegebenenfalls substituiert (durch Reste Phenyl, Hydroxy oder Amino), oder Dialkylamino, dessen Alkylteile zusammen mit dem Stickstoffatom, an das sie gebunden sind, einen Heterocyclus mit 5 oder 6 Ringgliedern bilden, der ein anderes Heteroatom enthalten kann, ausgewählt unter Sauerstoff, Schwefel oder Stickstoff, gegebenenfalls substituiert durch einen Rest Alkyl, Hydroxy, Hydroxyalkyl], oder substituiert durch Reste Amino, Alkylamino, Dialkylamino, dessen Alkylteile zusammen mit dem Stickstoffatom, an das sie gebunden sind, einen wie oben definierten Heterocyclus bilden können, oder auch einen Rest Cyclohexadienyl, Naphthyl, Indenyl oder den Rest eines gesättigten oder ungesättigten, mono- oder polycyclischen Heterocyclus mit 5 bis 9 Kohlenstoffatomen und einem oder mehreren Heteroatomen darstellt, ausgewählt unter Sauerstoff, Stickstoff oder Schwefel, und gegebenenfalls substituiert durch ein Halogenatom oder durch einen Rest Alkyl, Alkyloxy, Aminoalkyl, Alkylaminoalkyl oder Dialkylaminoalkyl,
- das Symbol R₂ ein Wasserstoffatom oder ein Halogenatom oder einen Rest Hydroxy, Alkyl, Aminoalkyl, Alkylaminoalkyl, Dialkylaminoalkyl, Alkyloxy, Alkylthio, Acyloxy, Carboxy, Alkyloxycarbonyl, Dialkylaminoalkyloxycarbonyl, Benzyloxycarbonyl, Amino oder Acylamino bedeutet, und
- das Symbol R₃ ein Phenylrest ist, gegebenenfalls substituiert in Position -2 durch einen Rest Alkyl oder Alkyloxy mit 1 oder 2 Kohlenstoffatomen,
wobei die oben genannten Reste Alkyl und Acyl (außer spezieller Erwähnung) gerade oder verzweigt sind und 1 bis 4 Kohlenstoffatomen enthalten, sowie die Salze, wenn sie existieren.

2. Perhydroisoindol-Derivat nach Anspruch 1, dadurch gekennzeichnet, daß das Symbol R₁ ein Rest eines gesättigten oder ungesättigten, mono- oder polycyclischen Heterocyclus ist, ausgewählt unter Thienyl, Furyl, Pyridyl, Dithiinyl, Indolyl, Isoindolyl, Benzothienyl, Thiazolyl, Isothiazolyl, Oxazolyl, Imidazolyl, Pyrrolyl, Triazolyl, Thiadiazolyl, Chinolyl, Isochinolyl oder Naphthyridinyl.

3. Perhydroisoindol-Derivat nach Anspruch 1, dadurch gekennzeichnet, daß es sich um 7,7-Diphenyl-4-(2-methoxyphenyl)-2-{[1-(2-dimethylamino-ethyl)-3-indolyl]-acetyl}-perhydroisoindol-4,5-diol-(3aR,4R,5R,7aR) handelt.

4. Perhydroisoindol-Derivat nach Anspruch 1, dadurch gekennzeichnet, daß es sich um 7,7-Diphenyl-4-(2-methoxyphenyl)-2-[(2-hydroxyphenyl)-acetyl]-perhydroisoindol-4,5-diol-(3aR,4R,5R,7aR) handelt.

5. Perhydroisoindol-Derivat nach Anspruch 1, dadurch gekennzeichnet, daß es sich um 7,7-Diphenyl-4-(2-methoxyphenyl)-2- [(2-benzyloxyphenyl)-acetyl]-perhydroisoindol-4,5-diol-(3aR,4R,5R,7aR) handelt.

6. Perhydroisoindol-Derivat nach Anspruch 1, dadurch gekennzeichnet, daß es sich um 7,7-Diphenyl-4-(2-methoxyphenyl)-2-[(3-indolyl)-acetyl]-perhydroisoindol-4,5-diol-(3aR,4R,5R,7aR) handelt.

7. Perhydroisoindol-Derivat nach Anspruch 1, dadurch gekennzeichnet, daß es sich um 7,7-Diphenyl-4-(2-methoxyphenyl)-2-[(5-hydroxy-3-indolyl)-acetyl]-perhydroisoindol-4,5-diol-(3aR,4R,5R, 7aR) handelt.

8. Verfahren zur Herstellung eines Perhydroisoindol-Derivates nach Anspruch 1, dadurch gekennzeichnet, daß man eine Säure oder ein reaktives Derivat der Säure der allgemeinen Formel 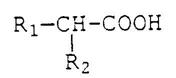 in der R₁ und R₂ wie in Anspruch 1 definiert sind, mit einem Isoindol-Derivat der allgemeinen Formel 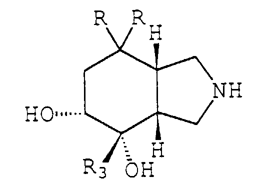 in der die Symbole R und R₃ wie in Anspruch 1 definiert sind, zur Reaktion bringt und anschließend das erhaltene Produkt gegebenenfalls in ein Salz umwandelt, wenn diese existieren.

9. Verfahren zur Herstellung eines Perhydroisoindol-Derivates nach Anspruch 1, worin R₁ ein durch Hydroxy substituierter Phenylrest ist, dadurch gekennzeichnet, daß man ein Perhydroisoindol-Derivat nach Anspruch 1, worin R₁ ein durch einen Benzyloxyrest substituierter Phenylrest ist, nach jeder bekannten Methode umwandelt, die den Rest des Moleküls nicht beeinflußt.

10. Verfahren zur Herstellung eines Perhydroisoindol-Derivates nach Anspruch 1, worin R₁ ein durch Amino substituierter Phenylrest ist, dadurch gekennzeichnet, daß man ein Perhydroisoindol-Derivat nach Anspruch 1, worin R₁ ein durch einen Nitrorest substituierter Phenylrest ist, nach jeder bekannten Methode zur Reduktion umwandelt, die den Rest des Moleküls nicht beeinflußt.

11. Verwendung von racemischen Derivaten (3aRS,4RS,5RS,7aRS), die den wie in Anspruch 1 definierten Perhydroisoindol-Derivaten entsprechen, zur Herstellung eines Arzneimittels für die Behandlung von Erkrankungen, die mit dem Rezeptor zu Tachykininen NK2 im Zusammenhang stehen.

12. Perhydroisoindol-Derivat, dadurch gekennzeichnet, daß es der allgemeinen Formel 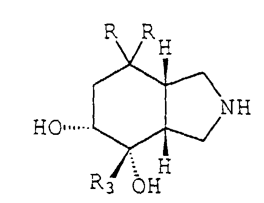 entspricht, in der R und R₃ wie in Anspruch 1 definiert sind, sowie seine Salze.

13. Synergistische Assoziationen von antagonistischen Wirkungen der Rezeptoren NK1 und NK2, bestehend aus einem Perhydroisoindol-Derivat nach einem der Ansprüche 1 oder 7 und einem für seine antagonistische Aktivität des Rezeptors NK1 bekannten Produkt.

14. Pharmazeutische Zusammensetzung, dadurch gekennzeichnet, daß sie mindestens ein Produkt nach Anspruch 1 enthält, in reinem Zustand oder in Form einer Assoziation mit einem oder mehreren kompatiblen und pharmazeutisch akzeptablen Zusatzstoffen oder Verdünnungsmitteln.

## Claims

1. A perhydroisoindole derivative, characterized in that it corresponds to the general formula: 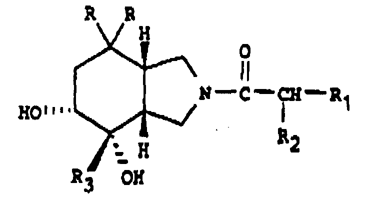 in which
- the symbols R are identical and represent phenyl radicals which are optionally substituted with a halogen atom or with a methyl radical in the 2- or 3-position,
- the symbol R₁ represents a phenyl radical which is optionally substituted with one or more halogen atoms or hydroxyl, benzyloxy or alkyl radicals which may optionally be substituted (with halogen atoms or with amino, alkylamino or dialkylamino radicals) or with alkyloxy or alkylthio radicals which may optionally be substituted [with hydroxyl, amino, alkylamino or dialkylamino radicals which are optionally substituted (with phenyl, hydroxyl or amino radicals), or dialkylamino radicals whose alkyl portions form, with the nitrogen atom to which they are attached, a 5- to 6-membered heterocycle which may contain another hetero atom chosen from oxygen, sulphur or nitrogen, and optionally substituted with an alkyl, hydroxyl or hydroxyalkyl radical], or substituted with amino, alkylamino or dialkylamino radicals whose alkyl portions may form, together with the nitrogen atom to which they are attached, a heterocycle as defined above, or represents a cyclohexadienyl, naphthyl or indenyl radical, or a saturated or unsaturated mono- or polycyclic heterocyclic radical containing 5 to 9 carbon atoms and one or more hetero atoms chosen from oxygen, nitrogen or sulphur, and optionally substituted with a halogen atom or with an alkyl, alkyloxy, aminoalkyl, alkylaminoalkyl or dialkylaminoalkyl radical,
- the symbol R₂ represents a hydrogen or halogen atom or a hydroxyl, alkyl, aminoalkyl, alkylaminoalkyl, dialkylaminoalkyl, alkyloxy, alkylthio, acyloxy, carboxyl, alkyloxycarbonyl, dialkylaminoalkyloxycarbonyl, benzyloxycarbonyl, amino or acylamino radical, and
- the symbol R₃ represents a phenyl radical which is optionally substituted in the 2-position with an alkyl or alkyloxy radical containing 1 or 2 carbon atoms,
the alkyl and acyl radicals cited above being (except where especially mentioned) straight or branched and containing 1 to 4 carbon atoms, as well as its salts when they exist.

2. A perhydroisoindole derivative according to claim 1, characterized in that the symbol R₁ is a saturated or unsaturated mono- or polycyclic heterocyclic radical chosen from thienyl, furyl, pyridyl, dithiinyl, indolyl, isoindolyl, benzothienyl, thiazolyl, isothiazolyl, oxazolyl, imidazolyl, pyrrolyl, triazolyl, thiadiazolyl, quinolyl, isoquinolyl or naphthyridinyl.

3. A perhydroisoindole derivative according to claim 1, characterized in that it is (3aR,4R,5R,7aR)-7,7-diphenyl-4-(2-methoxyphenyl)-2-[(1-(2-dimethylaminoethyl)-3-indolyl) acetyl]perhydro-4,5-isoindolediol.

4. A perhydroisoindole derivative according to claim 1, characterized in that it is (3aR,4R,5R,7aR)-7,7-diphenyl-4-(2-methoxyphenyl)-2-[(2-hydroxyphenyl)-acetyl]perhydro-4,5-isoindolediol.

5. A perhydroisoindole derivative according to claim 1, characterized in that it is (3aR,4R,5R,7aR)-7,7-diphenyl-4-(2-methoxyphenyl)-2- (2-benzyloxyphenyl)-acetyl]perhydro-4,5-isoindolediol.

6. A perhydroisoindole derivative according to claim 1, characterized in that it is (3aR,4R,5R,7aR)-7,7-diphenyl-4-(2-methoxyphenyl)-2-[(3-indolyl)acetyl]-perhydro-4,5-isoindolediol.

7. A perhydroisoindole derivative according to claim 1, characterized in that it is (3aR,4R,5R,7aR)-7,7-diphenyl-4-(2-methoxyphenyl)-2-[(5-hydroxy-3-indolyl)acetyl]perhydro-4,5-isoindolediol.

8. Process for the preparation of a perhydroisoindole derivative according to claim 1, characterized in that an acid or a reactive derivative of the acid of general formula: 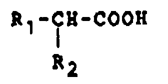 in which R₁ and R₂ are defined as in claim 1, is reacted with an isoindole derivative of general formula: 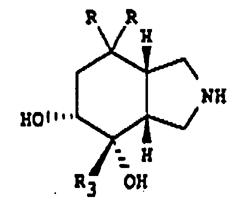 in which the symbols R and R₃ are defined as in claim 1, optionally followed by conversion of the product obtained to a salt, when they exist.

9. Process for the preparation of a perhydroisoindole derivative according to claim 1 for which R₁ is a phenyl radical which is substituted with a hydroxyl, characterized in that a perhydroisoindole derivative according to claim 1 for which R₁ is a phenyl radical which is substituted with a benzyloxy radical is converted by any known method which does not affect the remainder of the molecule.

10. Process for the preparation of a perhydroisoindole derivative according to claim 1 for which R₁ is an amino-substituted phenyl radical, characterized in that a perhydroisoindole derivative according to claim 1 for which R₁ is a phenyl radical which is substituted with a nitro radical is converted by any known reduction method which does not affect the remainder of the molecule.

11. Use of a (3aRS,4RS,5RS,7aRS) racemic derivative corresponding to the perhydroisoindole derivatives defined in claim 1 in the preparation of a medicament for the treatment of complaints mediated by the NK2 tachykinin receptor.

12. A perhydroisoindole derivative, characterized in that it corresponds to the general formula: 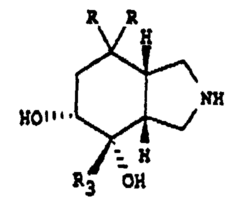 in which R and R₃ are defined as in claim 1, as well as its salts.

13. Combinations which synergize the antagonistic effects to NK1 and NK2 receptors, consisting of a perhydroisoindole derivative according to either of claims 1 or 7 and of a product known for its antagonistic activity to NK1 receptors.

14. Pharmaceutica**l** composition, characterized in that it contains at least one product according to claim 1, in the pure state or in the form of a combination with one or more compatible and pharmaceutically acceptable diluents or adjuvants.
